# EUROPEAN PATENT APPLICATION

(11) **EP 4 431 038 A1**
(43) Date of publication of application: **18.09.2024**
(21) Application number: 22891845.4
(22) Date of filing: 01.11.2022
(51) Int. Cl.: A61B 18/02

(54) **VACUUM WALL POSITION ADJUSTABLE CRYOABLATION NEEDLE**

(30) Priority: 11.11.2021 CN 202111329707
(71) Applicant: Accu Target Medipharma (Shanghai) Co., Ltd., Shanghai 201318 (CN)
(72) Inventor: YANG, Chi, Shanghai 201318 (CN); CHANG, Zhaohua, Shanghai 201318 (CN)
(74) Representative: EP&C
(86) International application number: PCT/CN2022/128871
(87) International publication number: WO 2023/083049

(57) **Abstract**

The present invention discloses a cryoablation needle with an adjustable vacuum wall position, including: a vacuum wall, a J-T slot and a vacuum wall adjusting apparatus, where the vacuum wall includes: a needle rod and an inner tube; the needle rod is provided with a needle tip at a distal end; the inner tube penetrates through the needle rod, and a cavity is formed between the inner tube and the needle rod; a first preset distance exists between a distal end of the inner tube and the distal end of the needle rod; the J-T slot penetrates through the inner tube; a distal end of the vacuum wall is switched between at least two adjusting positions relative to the J-T slot, the two adjusting positions including: a first adjusting position and a second adjusting position; in areas in which the vacuum wall is distributed in an axis direction of the vacuum wall, an area in which the cavity is located is a vacuum insulation area, and an area in which the first preset distance exists is a target area; when the needle tip is located at the first adjusting position, a distal end of the J-T slot is located in the target area; and when the needle tip is located at the second adjusting position, the distal end of the J-T slot is located in the vacuum insulation area. According to the present invention, the distal end of the vacuum wall is switched between the at least two adjusting positions, such that a cooling rate is greatly increased.

## Description

### TECHNICAL FIELD

The present invention relates to the technical field of cryoablation, and in particular to a cryoablation needle with an adjustable vacuum wall position.

### BACKGROUND

Cryoablation is a treatment approach that uses low temperature to destroy diseased tissues, which is considered to be an efficient and minimally invasive method to treat malignant tumors. The cryoablation is easy to operate, has few complications, and can effectively relieve pain. At the same time, ice balls formed by ablation have clear boundaries and are easy to observe, and lesions near large blood vessels or important organs can be safely ablated. The cryoablation may also adopt a multi-needle freezing manner, so that a wider ablation range is achieved, which is suitable for large lesions and irregular lesions.

In the process of cell freezing, ice crystals are firstly formed outside cells, which causes the concentration of extracellular solute to increase, resulting in a hypertonic environment, and water in the cells enters the outside of the cells, resulting in intracellular dehydration. The cells that lose water become shrunk and cell membranes are deformed, resulting in a "solution damage" in a high-concentration toxic environment. At the same time, ice crystals formed in the cells directly damage organelles and the cell membranes, causing further necrosis, commonly known as an "intracellular ice damage". The intracellular ice damage directly damages cell structures, and therefore is more destructive to the cells. Generally, the lower a cooling rate of the cells, the greater the probability of the "solution damage", and the higher the cooling rate, the easier it is to induce the "intracellular ice damage". Therefore, the higher cooling rate is generally pursued in the process of tumor cryoablation, which can kill tumors more thoroughly and greatly save surgery time.

The development of the cryoablation has undergone three stages. The first stage is a liquid nitrogen conveying and refrigeration technology, which conveys liquid nitrogen at -196°C to a needle tip of a cryoablation needle by a low driving pressure to achieve the purpose of cryoablation. Because a cold source of this technology completely relies on the liquid nitrogen, which is located in a main machine or liquid nitrogen barrel and has a long conveying distance from the needle tip, during the liquid nitrogen conveying process, only when a whole conveying pipeline reaches -196°C, temperature of the needle tip can reach -196°C. Therefore, the cooling rate of liquid nitrogen refrigeration is the lowest in the prior art. The second stage is a direct throttling refrigeration technology, which uses the principle of "Joule Thompson Effect" (J-T for short), and conveys ultra-high pressure gas at room temperature to the J-T slot (a capillary tube that produces the J-T effect) inside the cryoablation needle to directly throttle to produce low temperature. The cooling rate of this technology is relatively the highest in the prior art. However, structures such as the J-T slot and a finned tube inside the needle tip may consume a part of cold, thus prolonging cooling time. In addition, the ultra-high pressure gas used is not highly popularized and is expensive, resulting in difficulty in the promotion of this technology. The third stage is a pre-cooled throttling refrigeration technology, the principle of which is to pre-cool normal industrial gas that is at room temperature by a supporting main machine, and then convey pre-cooled normal industrial gas to the J-T slot inside the cryoablation needle to produce ablation temperature that is lower than preset temperature by throttling. This technology solves the problem that gas sources are expensive and scarce. Furthermore, this technology combines a throttling refrigeration technology, and thus the cooling rate thereof is obviously higher than that of the liquid nitrogen refrigeration technology, but is still lower than that of the direct throttling refrigeration technology.

### SUMMARY

For problems existing in the prior art, the present invention provides a cryoablation needle with an adjustable vacuum wall position, to resolve the problem of low cooling rate in the prior art.

In order to resolve the above technical problem, the present invention is implemented through the following technical solutions:

The present invention provides a cryoablation needle with an adjustable vacuum wall position, including a vacuum wall, a J-T slot and a vacuum wall adjusting apparatus, where
the vacuum wall includes: a needle rod and an inner tube;
the needle rod is provided with a needle tip at a distal end;
the inner tube penetrates through the needle rod, and a cavity is formed between the inner tube and the needle rod, the cavity being capable of forming a vacuum;
in an axis direction of the vacuum wall, a first preset distance exists between a distal end of the inner tube and the distal end of the needle rod; the distal end of the inner tube is an end of the inner tube close to the needle tip;
the J-T slot penetrates through the inner tube;
the needle tip is capable of being switched between at least two adjusting positions relative to the J-T slot, the at least two adjusting positions includes: a first adjusting position and a second adjusting position;needle tip
in areas in which the vacuum wall is distributed in the axis direction of the vacuum wall, an area in which the cavity is located is a vacuum insulation area, and an area in which the first preset distance exists is a target area;
when the needle tip is located at the first adjusting position, a distal end of the J-T slot is located in the target area; the distal end of the J-T slot is an end of the J-T slot close to the needle tip;
when the needle tip is located at the second adjusting position, the distal end of the J-T slot is located in the vacuum insulation area;
the vacuum wall adjusting apparatus is configured to enable the needle tip to be switched between the at least two adjusting positions by adjusting a position of the needle tip;
when the distal end of the J-T slot is located at the first adjusting position, in the axis direction of the vacuum wall, a second preset distance exists between the distal end of the J-T slot and the needle tip, and the second preset distance at least ensures that an ice ball formed by freezing is wrapped around the needle tip, that is, a refrigerant fluid returns from the inside of the target area and the inside of the vacuum insulation area after being sprayed from the J-T slot, where the refrigerant fluid exchanges heat with substances outside the whole target area during the process of returning from the inside of the target area; and
when the distal end of the J-T slot is located at the second adjusting position, in the axis direction of the vacuum wall, a third preset distance exists between the distal end of the J-T slot and a distal end of the vacuum insulation area, and the third preset distance at least ensures that the refrigerant fluid directly returns from the inside of the vacuum insulation area after being sprayed from the J-T slot. Only the relatively static refrigerant exists in the target area, which will not exchange heat with the substances outside the target area, that is, the refrigerant does not release any cold in the target area during a freezing process, the distal end of the vacuum insulation area being an end of the vacuum insulation area close to the needle tip.

Preferably, the vacuum wall adjusting apparatus includes: a first sliding block and a first sliding block guiding portion;
the first sliding block guiding portion is arranged in the axis direction of the vacuum wall;
the first sliding block is connected to the vacuum wall, and a position of the first sliding block guiding portion is fixed relative to the sliding block or the J-T slot;
if the position of the first sliding block guiding portion is fixed relative to the sliding block, the first sliding block guiding portion and the J-T slot are capable of sliding relatively; if the position of the first sliding block guiding portion is fixed relative to the J-T slot, the first sliding block and the first sliding block guiding portion are capable of sliding relatively; and
the first sliding block and the vacuum wall are capable of being controlled to synchronously move in the axis direction, to switch the needle tip between the adjusting positions.

Preferably, the cryoablation needle with the adjustable vacuum wall position further includes: a first sealing assembly, where the first sealing assembly is hermetically connected to a proximal end of the inner tube, the proximal end of the inner tube being an end of the inner tube far away from the needle tip; and
a dynamic seal is formed between the first sealing assembly and the first sliding block.

Preferably, the first sealing assembly includes: a sealing ring, a sealing slot and a sealing press piece, where
a distal end of the sealing slot is hermetically fixed to the proximal end of the inner tube, the distal end of the sealing slot being an end of the sealing slot close to the needle tip;
the sealing ring is arranged between the sealing press piece and the first sliding block, and the sealing press piece is arranged between the sealing ring and the sealing slot;
the sealing ring and the sealing press piece are slidably connected to the sealing slot; and
the first sliding block, the sealing ring, and the sealing press piece are capable of being controlled to synchronously move in the axis direction.

Preferably, the cryoablation needle with the adjustable vacuum wall position further includes: a spring and a clamping piece, where
one end of the spring moves synchronously with the distal end of the J-T slot, and is further connected to the clamping piece; the clamping piece is capable of entering and exiting from a clamped position;
the other end of the spring is fixed relative to the J-T slot;
when the clamping piece is located at the clamped position, the spring is limited by the clamping piece to keep in a deformation state, and a distal end of the vacuum wall is located at the second adjusting position;
the deformation state is a compression state or a tension state; and
when the clamping piece exits from the clamped position, the spring is capable of generating a restoring force for restoring from the deformation state to a natural state, and the restoring force is capable of driving the distal end of the vacuum wall to enter the first adjusting position from the second adjusting position.

Preferably, the cryoablation needle with the adjustable vacuum wall position further includes: a first handle, wherein the first handle includes: a front handle section and a rear handle section;
the front handle section is fixedly connected to the vacuum wall;
a distal end of the rear handle section is inserted into a proximal end of the front handle section, and the two are capable of sliding relatively; and
the front handle section and/or the rear handle section is provided with a limiting ring, and the limiting ring is configured to limit the furthest distance of the vacuum wall moving toward the distal end.

Preferably, the clamping piece includes: a hand-held portion and a C-shaped ring, where
the hand-held portion is arranged on the C-shaped ring; and
the C-shaped ring is wrapped around an outer wall of the rear handle section.

Preferably, the vacuum wall includes: a front vacuum wall section and a rear vacuum wall section, from a distal end to a proximal end of the vacuum wall, the front vacuum wall section and the rear vacuum wall section are sequentially distributed, and the front vacuum wall section and the rear vacuum wall section are capable of moving relatively; the needle tip is located at the front vacuum wall section;
the vacuum wall adjusting apparatus enables the needle tip to be switched between the at least two adjusting positions by adjusting the relative position of the front vacuum wall section and the rear vacuum wall section.

Preferably, the vacuum wall adjusting apparatus includes: a second sliding block and a second sliding block guiding portion;
the second sliding block guiding portion is arranged in the axis direction of the vacuum wall;
the second sliding block is connected to the front vacuum wall section, and a position of the second sliding block guiding portion is fixed relative to the front vacuum wall section or the rear vacuum wall section;
if the position of the second sliding block guiding portion is fixed relative to the rear vacuum wall section, the second sliding block and the second sliding block guiding portion are capable of sliding relatively; if the position of the second sliding block guiding portion is fixed relative to the front vacuum wall section, the rear vacuum wall section and the second sliding block are capable of sliding relatively; and
the second sliding block and the front vacuum wall section are capable of being controlled to synchronously move in the axis direction, to switch the needle tip between the adjusting positions.

Preferably, the cryoablation needle with the adjustable vacuum wall position further includes: a second sealing assembly, where the second sealing assembly is arranged between the front vacuum wall section and the rear vacuum wall section, and is configured to form a dynamic seal between the front vacuum wall section and the rear vacuum wall section.

Preferably, the cryoablation needle with the adjustable vacuum wall position further includes: a shifting block and a second handle, where the second handle includes: a handle adjusting slot;

the shifting block is connected to the sliding block, the shifting block is arranged in the handle adjusting slot, and the shifting block extends out of an outer wall of the second handle;
the shifting block is slidably connected to the handle adjusting slot; and
theshifting block and the second sliding block are capable of being controlled to synchronously move in the axis direction.

Preferably, the vacuum wall further includes: an outer tube and a gasket, where
a distal end of the outer tube is hermetically connected to a proximal end of the needle rod, a proximal end of the outer tube is hermetically connected to the proximal end of the inner tube, the distal end of the outer tube is an end of the outer tube close to the needle tip, and the proximal end of the outer tube is an end of the outer tube far away from the needle tip;
the gasket is arranged between an outer wall of the inner tube and an inner wall of the needle rod to form a sealed connection;
from the distal end to the proximal end of the inner tube, the inner tube sequentially includes: an inner tube front section and an inner tube rear section;
the inner tube front section penetrates through the needle rod; the inner tube rear section penetrates through the outer tube; and
if the vacuum wall includes: a front vacuum wall section and a rear vacuum wall section, the front vacuum wall section includes the needle rod and the inner tube front section, and the rear vacuum wall section includes the outer tube and the inner tube rear section.

Preferably, an outer diameter of the outer tube is greater than an outer diameter of the needle rod, and an inner diameter of the outer tube is greater than an inner diameter of the needle rod; and
an outer diameter of the inner tube rear section is greater than an outer diameter of the inner tube front section; an inner diameter of the inner tube rear section is greater than an inner diameter of the inner tube front section.

Preferably, the cryoablation needle with the adjustable vacuum wall position further includes: a temperature measuring wire, where
a distal end of the temperature measuring wire is a temperature measuring point; the distal end of the temperature measuring wire is an end of the temperature measuring wire close to the needle tip; and
thetemperature measuring point is arranged at the distal end of the J-T slot, and used for measuring temperature at the distal end of the J-T slot.

Compared with the prior art, the present invention has the following advantages:
(1) According to the cryoablation needle with the adjustable vacuum wall position provided in the present invention, the vacuum wall is adjustable in position, and at least includes the two adjusting positions, and freezing is started after the J-T slot is returned to the inside of the vacuum insulation area, so that all conveying pipelines at a main machine side and a cryoablation needle side can be pre-purged (cooled), and no cold is consumed at the target area during a pre-purging process. Therefore, all cold is used for cooling the conveying pipelines, and thus the rate of this conveying pipeline cooling process is the highest. Furthermore, no cold is released at the target area during the pre-purging process, so that there are no frosting and freezing phenomena in the target area, and then formal surgery can be performed directly.
(2) According to the cryoablation needle with the adjustable vacuum wall position provided in the present invention, the distal end of the vacuum wall is adjustable in position, and at least includes the two adjusting positions, only the target area of the vacuum wall of the pre-purged cryoablation needle is not cooled, and after the J-T slot is returned to the inside of the target area, all heat loads only come from the target area and tumor tissues outside the target area. Therefore, the cooling rate of this freezing process can be greatly increased.
(3) According to the cryoablation needle with the adjustable vacuum wall position provided in the present invention, after a needling test procedure ends, a pre-purging mode can be kept enabled, the inside (the distal end of the J-T slot) of the vacuum insulation area is kept at the lowest temperature. Since the target area does not release any cold, operations such as puncturing, scanning and positioning can be performed, and then the cryoablation needle is adjusted to a freezing mode after the puncturing is in place. In this case, the inside of the target area is directly reduced from the room temperature to the lowest temperature instantly. Therefore, ultimate rapid cooling of the formal surgery can be achieved.
(4) The cryoablation needle with the adjustable vacuum wall position provided in the present invention is wide in application range, and can be applied to all existing cryoablation technologies: liquid nitrogen conveying and refrigeration technology, direct throttling refrigeration technology and pre-cooled throttling refrigeration technology. The cryoablation needle with the adjustable vacuum wall position is not only applicable to percutaneous puncture cryoablation instruments, but also applicable to cryoablation instruments of natural orifice transluminal surgery.

Certainly, implementing any product of the present invention does not necessarily need to simultaneously achieve all the advantages described above.

### BRIEF DESCRIPTION OF DRAWINGS

In order to explain the technical solutions in the embodiments of the present invention or in the prior art more clearly, the drawings used in the description of the embodiments or the prior art will be briefly introduced below. Obviously, the drawings in the following description are merely some embodiments of the present invention. For those of ordinary skill in the art, other drawings can be obtained based on these drawings without creative efforts.
FIG. 1 is a diagram of a vacuum wall adjustment principle of a hard cryoablation needle with an adjustable vacuum wall position according to an embodiment of the present invention;
FIG. 2 is a diagram of a vacuum wall adjustment principle of a flexible cryoablation needle with an adjustable vacuum wall position according to an embodiment of the present invention;
FIG. 3 is a diagram of a pre-purging mode of a vacuum wall overall adjusting solution for a hard cryoablation needle according to a preferred embodiment of the present invention;
FIG. 4 is a diagram of a freezing mode of a vacuum wall overall adjusting solution for a hard cryoablation needle according to a preferred embodiment of the present invention;
FIG. 5 is a schematic diagram of a vacuum wall of a flexible cryoablation needle according to a preferred embodiment of the present invention;
FIG. 6 is a diagram of a pre-purging mode of a vacuum wall overall adjusting solution for a flexible cryoablation needle according to a preferred embodiment of the present invention;
FIG. 7 is a diagram of a freezing mode of a vacuum wall overall adjusting solution for a flexible cryoablation needle according to a preferred embodiment of the present invention;
FIG. 8 is a schematic diagram of a sliding block according to a preferred embodiment of the present invention;
FIG. 9 is a schematic diagram of a clamping piece according to a preferred embodiment of the present invention;
FIG. 10 is a diagram of a pre-purging mode of a front vacuum wall section adjusting solution for a hard cryoablation needle according to a preferred embodiment of the present invention; and
FIG. 11 is a diagram of a freezing mode of a front vacuum wall section adjusting solution for a hard cryoablation needle according to a preferred embodiment of the present invention.

### Description of reference numerals:

1 - J-T slot,
21 - needle rod,
211 - needle tip,
22 - inner tube,
221 - inner tube front section,
222 - inner tube rear section,
23 - outer tube,
24 - gasket,
25 - target area,
26 - vacuum insulation area,
28 - vacuum tee,
281 - tee connecting portion,
282 - tee bypass,
291 - vacuum connecting tube,
292 - vacuum hose,
293 - return gas connecting tube,
294 - shunt,
3 - mandrel,
4 - finned tube,
51 - sealing ring,
52 - sealing slot,
53 - sealing press piece,
6 - gas intake tube,
7 - gas return tube,
8 - sliding block,
83 - middle fixing hole,
85 - gas intake/return tube fixing hole,
86 - shifting block,
9 - handle,
92 - front handle section,
921 - front adjusting section,
922 - front limiting ring,
93 - rear handle section,
931 - rear adjusting section,
932 - rear limiting ring,
933 - sliding block fixing hole,
934 - shunt fixing hole,
935 - hose guiding tube,
94 - handle adjusting slot,
10 - clamping piece,
101 - hand-held portion,
103 - C-shaped ring,
120 - spring,
13 - outer sleeve,
14 - temperature measuring wire,
141 - temperature measuring point.

### DESCRIPTION OF EMBODIMENTS

The technical solutions in embodiments of the present invention will be clearly and fully described in combination with the drawings of the embodiments of the present invention; it is obvious that the described embodiments are only a part of, and not all embodiments of, present invention. Based on the embodiments of the present invention, all other embodiments obtained by those of ordinary skill in the art without creative efforts should fall within the protection scope of the present invention.

In the description of the specification of the present invention, it should be understood that the term "upper portion", "lower portion", "upper end", "lower end", "upper surface", "lower surface" or the like indicates an orientation or positional relationship based on that shown in the drawings, which is merely for ease of description and simplicity of description, and is not intended to indicate or imply that the apparatus or element referred to must have a particular orientation, be constructed and operate in a particular orientation, and therefore cannot be construed as a limitation on the present invention.

In the description of the specification of the present invention, the terms "first" and "second" are used for descriptive purposes only, and cannot be construed as indicating or implying relative importance or implicitly indicating the number of technical features indicated. Therefore, a feature defined with "first" and "second" may explicitly or implicitly include one or more of the features.

In the description of the present invention, "a plurality of" means multiple, such as two, three, four or the like, unless specifically defined otherwise.

The technical solutions of the present invention will be described in detail below by specific embodiments. The following several specific embodiments may be mutually combined, and same or similar concepts or processes may not be repeatedly described in some embodiments.

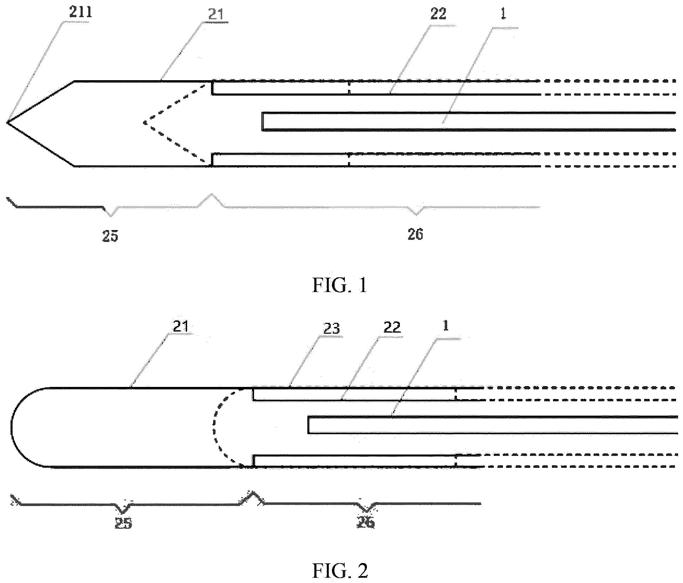

As shown in FIG. 1 and FIG. 2, which each is a diagram of a J-T slot adjustment principle of a cryoablation needle with an adjustable vacuum wall position according to an embodiment of the present invention.

Referring to FIG. 1 and FIG. 2, the cryoablation needle with the adjustable vacuum wall position according to this embodiment includes: a vacuum wall, a J-T slot 1 and a vacuum wall adjusting apparatus. The vacuum wall includes: a needle rod 21 and an inner tube 22, and the needle rod 21 is provided with a needle tip 211 at a distal end. The inner tube 22 penetrates through the needle rod 21, and a cavity is formed between the inner tube 22 and the needle rod 21, the cavity is a cavity that can form a vacuum, which may be a permanent vacuum cavity or a real-time vacuum cavity. The vacuum cavity plays a role in heat insulation and preventing frostbite of normal tissues.

A first preset distance (the first preset distance can be understood as a spacing distance in an axis direction of the vacuum wall) exists between a distal end of the inner tube 22 and the distal end of the needle rod. The distal end of the inner tube 22 is an end of inner tube 22 close to the needle tip 211. The J-T slot 1 penetrates through the inner tube 22.

In areas in which the vacuum wall is distributed in the axis direction of the vacuum wall, since the vacuum cavity plays a role in heat insulation, an area in which the cavity is located is a vacuum insulation area 26, and an area in which the first preset distance exists is a target area 25.

The distal end of the vacuum wall has at least two adjusting positions: a first adjusting position and a second adjusting position, so that the distal end of the vacuum wall can be switched between the at least two adjusting positions relative to the J-T slot (for example, switching can be realized by moving in the axis direction of the vacuum wall), the at least two adjusting positions including: a first adjusting position and a second adjusting position. When the distal end of the vacuum wall is located at the first adjusting position, the distal end of the J-T slot 1 is located in the target area 25, and it can be understood as being in a freezing mode, as shown by dashed lines in FIG. 1. The distal end of the J-T slot 1 is an end of the J-T slot 1 close to the needle tip 211. When the distal end of the vacuum wall is located at the second adjusting position, the distal end of the J-T slot 1 is located in the vacuum insulation area 26, and it can be understood as being in a pre-purging mode, as shown by solid lines in FIG. 1.

The vacuum wall adjusting apparatus is configured to enable the distal end of the vacuum wall to be switched between the at least two adjusting positions. Specifically, the distal end of the vacuum wall can be adjusted in response to external manipulation, to be switched between the at least two adjusting positions. Furthermore, any apparatus that can realize this switching adjustment function does not depart from the description of this solution.

When the distal end of the vacuum wall is located at the first adjusting position, a second preset distance exists between the distal end of the J-T slot 1 and the needle tip 211, and the second preset distance at least ensures that an ice ball formed by freezing is wrapped around the needle tip. That is, a refrigerant fluid returns from the inside of the target area 25 and the inside of the vacuum insulation area 26 after being sprayed from the J-T slot 1, where the refrigerant fluid exchanges heat with substances outside the whole target area during the process of returning from the inside of the target area 25.

When the distal end of the vacuum wall is located at the second adjusting position, a third preset distance exists between the distal end of the J-T slot 1 and a distal end of the vacuum insulation area, and the third preset distance at least ensures that the refrigerant fluid directly returns from the inside of the vacuum insulation area after being sprayed from the J-T slot 1. Only a relatively static refrigerant exists in the target area, which will not exchange heat with the substances outside the target area. That is, the refrigerant does not release any cold in the target area during a freezing process. The distal end of the vacuum insulation area 26 is an end of the vacuum insulation area close to the needle tip 211.

When the distal end of the vacuum wall is located at the second adjusting position, the refrigerant fluid may have a certain spraying distance when being sprayed from the J-T slot 1. In order to ensure that the refrigerant fluid will not be sprayed to the target area after being sprayed from the J-T slot, a distance between the distal end of the J-T slot and the distal end of the vacuum insulation area is the third preset distance, the third preset distance being greater than or equal to the spraying distance of the refrigerant fluid. In this way, it can be ensured that the refrigerant fluid will not be sprayed to the target area after being sprayed from the J-T slot 1, and can directly return from the inside of the vacuum insulation area, thus ensuring that only the relatively static refrigerant exists in the target area.

The first preset distance, the second preset distance and the third preset distance may be understood as spacing distances in the axis direction of the vacuum wall. In addition, an axial direction mentioned later can be understood as the axis direction of the vacuum wall.

In an embodiment, the vacuum wall is a hard-material vacuum wall, which can be applied to percutaneous cryoablation instruments. As shown in FIG. 1, the needle tip 211 is in the form of a pinpoint.

In an embodiment, the vacuum wall is a flexible-material vacuum wall, which can be applied to natural orifice transluminal ablation instruments. As shown in FIG. 2, the needle tip 211 is in the form of a circular arc. Preferably, the needle rod 21 and the inner tube 22 may be made of flexible nonmetallic materials or freely bendable metallic materials, such as PTFE or PTFE braided tubes or stainless-steel bellows.

In an embodiment, a use process of the cryoablation needle with the adjustable vacuum wall position is as follows: Before surgery, the cryoablation needle in the pre-purging mode is taken out to be mutually connected to a main machine. The needle rod 21 (at least the target area 25) of the cryoablation needle is inserted into physiological saline. A needling test function is enabled. A rewarming operation is firstly performed during needling test. When the temperature of the needle tip rises to a certain temperature value within a certain time, it proves that a rewarming function is normal. Then, a program automatically performs a freezing operation. When the temperature of the needle tip is reduced to a certain temperature value within a certain time, it proves that a freezing function is normal. In this case, the time the needle tip is kept at the lowest temperature can be properly prolonged for sufficient pre-purging, and then the needling test is automatically stopped. During the freezing operation, whether there is a frosting phenomenon in the vacuum insulation area 26 is observed. If there is no frosting phenomenon, it proves that a heat insulation function is normal. Whether there is air leakage in the needle tip immersed in the physiological saline is observed during the whole process. If there is not air leakage, it proves that gas tightness is normal. After the needling test, conveying pipelines of both the main machine and the cryoablation needle have been pre-purged (cooled). Then, the freezing function can be enabled (or a separately configured pre-purging function can be enabled) at first, and freezing at this stage can be carried out at a lower working pressure, or gas can be intermittently introduced, so that the temperature at the distal end of the J-T slot can be kept at the lowest temperature while gas consumption can be reduced. Next, under the condition of keeping the freezing function enabled, percutaneous puncturing can be performed under the guidance of imaging, so that the needle tip can reach an expected tumor position. In this case, the distal end of the vacuum wall can be adjusted to move toward a proximal end, and stop at the first adjusting position, to switch to the freezing mode. Since the whole conveying pipeline is already in a low temperature state, a cooling heat load of the cryoablation needle only exists in the target area 25 and tumor tissues outside the target area. Therefore, after switching to the freezing mode, the distal end of the J-T slot can still be kept at the lowest temperature, and an outer wall of the target area 25 will be reduced from room temperature to below -100°C instantly. In this way, surgical time for ablating a tumor with the same size is shortened, or a larger ablation range (ice ball) is produced within the same time. In addition, due to more rapid cooling of the tumor tissues, the probability of intracellular ice damage of tumor cells is greatly increased, and then freezing damage of the tumor cells is more thorough and the ablation effect is better.

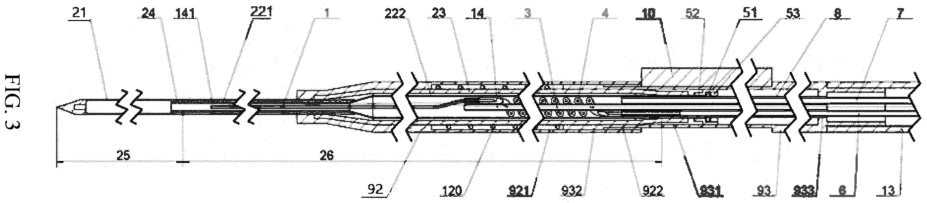

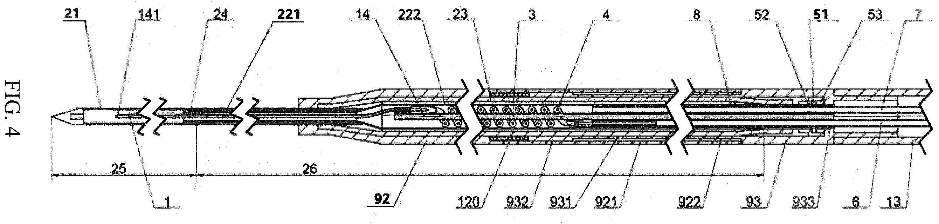

In a preferred embodiment, the position adjustment of the distal end of the vacuum wall adopts a vacuum wall overall adjusting (with the J-T slot 1 fixed) mode. Refer to FIG. 3 and FIG 4.

In an embodiment, the vacuum wall adjusting apparatus may include: a mandrel 3 and a sliding block 8. The mandrel 3 is located in an axial direction of the vacuum wall. The sliding block 8 is connected to the vacuum wall. The sliding block is located at a proximal end of the inner tube. The sliding block 8 is configured to drive the mandrel 3 to move in the axial direction, to drive the vacuum wall to move in the axial direction, so that the distal end of the vacuum wall is switched between the at least two adjusting positions. It can be seen that the sliding block 8 and the vacuum wall can be controlled to synchronously move in the axis direction.

As shown in FIG. 3, the distal end of the vacuum wall is located at the second adjusting position, that is, the diagram of the pre-purging mode. As shown in FIG. 4, the distal end of the vacuum wall is located at the first adjusting position, that is, the diagram of the freezing mode.

In the foregoing embodiment, the mandrel 3 is a first sliding block guiding portion. The sliding block 8 is arranged on an outer wall of the mandrel 3. The mandrel 3 guides the sliding block 8 to move in the axis direction. In a different embodiment, the vacuum wall may be arranged on the outer wall of the mandrel 3. The sliding block 8 may be arranged on the outer wall of the vacuum wall. The sliding block 8 moves, and then drives the vacuum wall to move along the mandrel 3. In a different embodiment, a guiding tube arranged in the axis direction of the vacuum wall can also be used for guiding. The guiding tube is arranged in the axis direction. The sliding block 8 is arranged on an inner wall of the guiding tube, slides along the guiding tube, and can also be guided to slide in the axis direction. In a different embodiment, the first sliding block guiding portion may be connected to a component to be adjusted (the vacuum wall). The sliding block is connected to the first sliding block guiding portion. The first sliding block guiding portion is arranged in the axis direction, and can move in the axis direction. The sliding block 8 drives the first sliding block guiding portion to move in the axis direction, and then drives the vacuum wall to move in the axis direction.

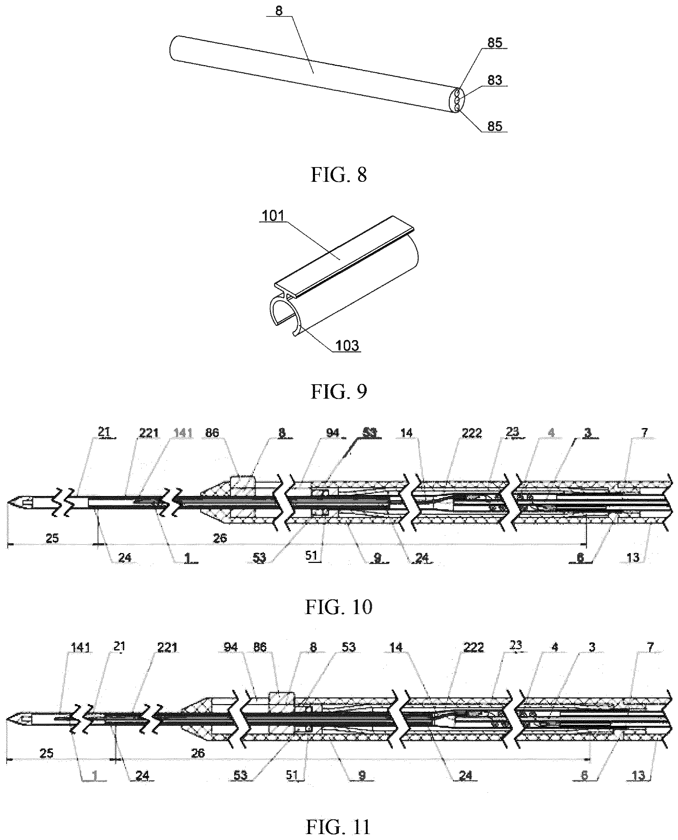

In an embodiment, the sliding block 8 includes: a middle fixing hole 83 and two gas intake/return tube fixing holes 85. Refer to FIG. 8. The three holes may be arranged in a straight line, or may be arranged in a triangular shape, or may be arranged in different modes according to actual requirements. The mandrel 3, a gas intake tube 6 and a gas return tube 7 are respectively inserted into the middle fixing hole 83 and the two gas intake/return tube fixing holes 85 and hermetically connected to the same. The gas intake tube 6 and the gas return tube 7 are in communication with the J-T slot. Intake gas of the J-T slot enters from the gas intake tube 6, and return gas of the J-T slot exits from the gas return tube 7.

In an embodiment, on the basis of the foregoing embodiment of adjusting the vacuum wall integrally, in order to prevent gas in the vacuum wall from leaking during the process of adjusting a position of the distal end of the vacuum wall, the cryoablation needle with the adjustable vacuum wall position further includes a sealing assembly. Refer to FIG. 2 and FIG. 3. The sealing assembly is hermetically connected to the proximal end of the inner tube 22. The proximal end of the inner tube 22 is an end of the inner tube far away from needle tip 211. A dynamic seal is formed between the sealing assembly and the sliding block 8.

Furthermore, the sealing assembly includes: a sealing ring 51, a sealing slot 52 and a sealing press piece 53. A distal end of the sealing slot 52 is fixed to and sealed with the proximal end of the inner tube 22. The distal end of the sealing slot 52 is an end of the sealing slot close to the needle tip 211. The sealing ring 51 is placed in the sealing slot 52. The sealing press piece 53 is screwed into the sealing slot 52 in the axial direction, to fix the sealing ring 51 between the sealing slot 52 and the sealing press piece 53. The mandrel 3 is inserted into the sealing ring 51 and the sealing press piece 53, so that the sealing ring 51 is radially extruded and deformed between the mandrel 3 and the sealing slot 52 to form a dynamic seal. Optionally, the sealing ring 51 may be a rubber sealing ring, such as a Buna-N rubber O-shaped ring, or may be a low-temperature-resistant Variseal sealing ring including fluoropolymer and a metal spring.

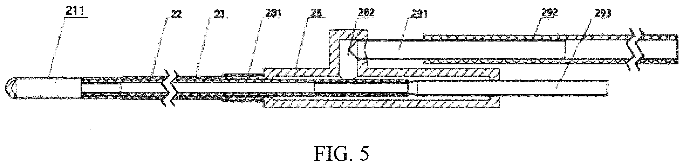

In a preferred embodiment, the vacuum wall of the flexible cryoablation needle may further include: a vacuum tee 28, a vacuum connecting tube 291, a vacuum hose 292 and a return gas connecting tube 293. Refer to FIG. 5. A proximal end of the inner tube 22 is connected to and sealed with a distal end of the return gas connecting tube 293. A proximal end of the outer tube 23 is connected to and sealed with a tee connecting portion 281. A proximal end of the vacuum tee 28 is connected to and sealed with the return gas connecting tube 293. A distal end of the vacuum connecting tube 291 is inserted into a tee bypass 282. The vacuum hose 292 is inserted into the vacuum connecting tube 291. By vacuum-pumping a proximal end of the vacuum hose 292, a gap between the inner tube 22 and the outer tube 23 can be kept in a vacuum state, to prevent frostbite of a normal natural cavity wall.

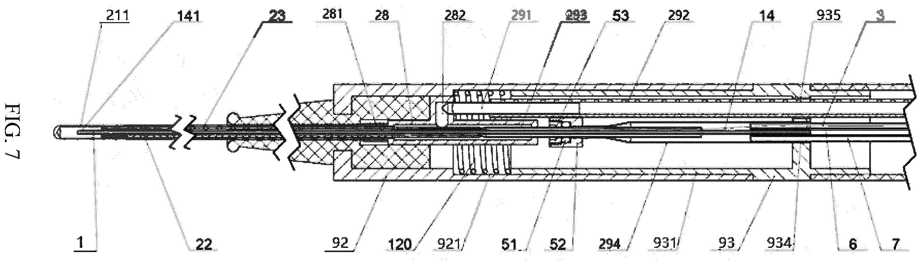

In an embodiment, the cryoablation needle with the adjustable vacuum wall position further includes: a shunt 294, configured to seal gaps between the gas intake tube 6, the gas return tube 7 and the mandrel 3. The gas intake tube 6, the gas return tube 7 and the mandrel 3 are inserted into a proximal end of the shunt 294 for sealing. Refer to FG. 6 and FIG. 7.

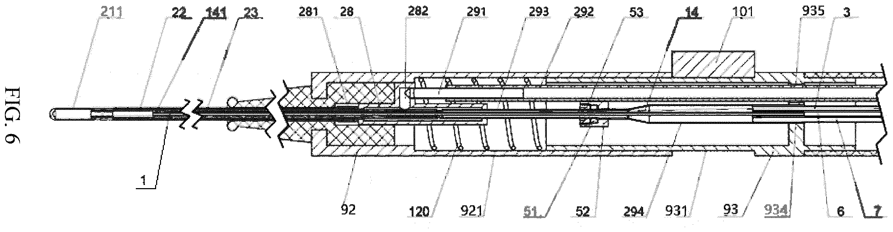

In an embodiment, on the basis of the foregoing embodiment of adjusting the vacuum wall integrally, the position adjustment of the distal end of the vacuum wall may be achieved by manual forward and backward adjustment, or may be achieved by a prefabricated spring 120, as shown in FIG. 3, FIG. 4, FIG. 6 and FIG. 7. The spring 120 is configured to keep the position of the distal end of the vacuum wall. When the spring 120 is in a compression or tension state, the distal end of the vacuum wall is located at the second adjusting position (pre-purging mode), as shown in FIG. 3 and FIG. 6, which take the spring in the tension state as an example. When the spring is in a natural state, the distal end of the vacuum wall is located at the first adjusting position (freezing mode), as shown in FIG. 4 and FIG. 7. Furthermore, the cryoablation needle with the adjustable vacuum wall position further includes a clamping piece 10, as shown in FIG. 3, FIG. 4, FIG. 6 and FIG. 7. When the distal end of the vacuum wall is located at the second adjusting position, the clamping piece is configured to keep the spring 120 in the tension state. When the distal end of the vacuum wall needs to be switched to the first adjusting position, only the clamping piece 10 needs to be pulled out.

In a different embodiment, when the distal end of the vacuum wall is located at the second adjusting position, the spring can also be in the compression state. When the distal end of the vacuum wall is located at the first adjusting position, the spring is in the natural state.

In an embodiment, in order to facilitate grasping and adjustment, the cryoablation needle with the adjustable vacuum wall position further includes: a handle 9. The handle includes: a front handle section 92 and a rear handle section 93. Refer to FIG. 3 and FIG. 4. The front handle section includes: a front adjusting section 921 and a front limiting ring 922. The rear handle section 93 includes: a rear adjusting section 931, a rear limiting ring 932 and a sliding block fixing hole 933. The front handle section 92 is fixedly connected to the vacuum wall. The sliding block 8 is fixedly connected to the rear handle section 93 through the sliding block fixing hole 933. The rear adjusting section 931 is inserted into the front adjusting section 921. The front limiting ring 922 and the rear limiting ring 932 limit each other, to limit the position adjustment of the distal end of the vacuum wall. When the vacuum wall moves to the second adjusting position, the front limiting ring 922 and the rear limiting ring 932 limit each other, to limit the vacuum wall from further moving toward the distal end (that is, to limit the furthest distance of the vacuum wall moving toward the distal end).

In a different embodiment, the front handle section 92 and the rear handle section 93 may not include the front limiting ring 922 and the rear limiting ring 932. Refer to FIG. 6 and FIG. 7. Limitation can be performed through a proximal end of the front handle section 92 and a distal end of the rear handle section 93.

In a preferred embodiment, referring to FIG. 3 and FIG. 6, a fixing mode of the clamping piece 10 is as follows: a distal end of the spring 120 is fixedly connected to a distal end of the front adjusting section 921, a proximal end of the spring 120 is fixedly connected to the rear limiting ring 932 (that is, being fixed relative to the sliding block 8), and the clamping piece 10 is connected to the distal end of the spring 120. The mode of inserting the rear adjusting section 931 into the front adjusting section 921 is adopted. The rear adjusting section 931 is thin. In the pre-purging mode, a section of the rear adjusting section 931 (between a proximal end of the front limiting ring 922 and a proximal end of the rear adjusting section 931) is exposed, and the clamping piece 10 can just be clamped into this exposed section of the rear adjusting section 931, thus fixing the relative position of the sliding block 8 and the vacuum wall. In this case, the spring 120 is in the tension state, that is, the current pre-purging mode is kept. When it is necessary to switch to the freezing mode from the pre-purging mode, the rear handle section 93 can be held by a hand and the clamping piece 10 can be pulled out. Under a tensile force of the spring 120, the sliding block 8 drives the front handle section 92, that is, driving the vacuum wall to move toward a proximal end, until the proximal end of the vacuum wall is propped against the rear handle section 93 (a distal end of the sliding block fixing hole 933). In this case, the vacuum wall stops at the first adjusting position, and is switched to the freezing mode. Refer to FIG. 4 and FIG. 7.

In an embodiment, when the cryoablation tube is flexible and includes the shunt 294, the rear adjusting section 931 of the handle is further provided with a shunt fixing hole 934 for the shunt 294 to pass through, by which the shunt is radially limited. Refer to FIG. 6 and FIG. 7.

In an embodiment, when the cryoablation tube is flexible and includes the vacuum hose 292, the rear adjusting section 931 of the handle is further provided with a hose guiding tube 935 for the vacuum hose 292 to pass through, by which the direction of the vacuum hose is guided. Refer to FIG. 6 and FIG. 7.

In a preferred embodiment, in order to facilitate the fixing of the clamping piece and the insertion and removal adjustment of the clamping piece, the clamping piece 10 includes: a hand-held portion 101 and a C-shaped ring 103. Refer to FIG. 9. The hand-held portion 101 is arranged on the C-shaped ring 103, which is convenient for grasping and adjustment. The C-shaped ring 103 is wrapped around an outer wall of the handle 9, which can prevent the clamping piece from falling off radially.

In a preferred embodiment, in order to improve a heat dissipation function, the cryoablation needle with the adjustable vacuum wall position further includes: a finned tube 4. The finned tube 4 is arranged on an outer wall of the mandrel 3. Refer to FIG. 3 and FIG. 4.

In a preferred embodiment, on the basis of the foregoing embodiment of adjusting the vacuum wall integrally, in order to increase the internal volume of the proximal end of the inner tube, for example, the finned tube 4 may be inserted into the proximal end of the inner tube, or more other components can be accommodated. Since the internal volume of the proximal end of the inner tube needs to be increased, the internal volume of the proximal end of the vacuum wall also needs to be increased. The vacuum wall further includes: an outer tube 23 and a gasket 24. Refer to FIG. 3 and FIG. 4. The gasket 24 is arranged between an outer wall of the distal end of the inner tube 22 and an inner wall of the needle rod 21, to form a sealed connection. A distal end of the outer tube 23 is hermetically connected to a proximal end of the needle rod 21, and the proximal end of the outer tube 23 is hermetically connected to the proximal end of the inner tube 22. An outer diameter of the outer tube 23 is greater than an outer diameter of the needle rod 21. An inner diameter of the outer tube 23 is greater than an inner diameter of the needle rod. The distal end of the outer tube 23 is an end of the outer tube 23 close to the needle tip 211. The proximal end of the outer tube 23 is an end of the outer tube 23 far away from the needle tip 211. Furthermore, from the distal end to the proximal end of the inner tube 22, the inner tube 22 sequentially includes: an inner tube front section 221 and an inner tube rear section 222. An outer diameter of the inner tube rear section 222 than an outer diameter of the inner tube front section 221. An inner diameter of the inner tube rear section 222 is greater than an inner diameter of the inner tube front section 221. The inner tube front section 221 is located inside the needle rod 21. The inner tube rear section 222 is located inside the outer tube 23.

In a preferred embodiment, the position adjustment of the distal end of the vacuum wall can be achieved by adopting the mode of dividing the vacuum wall into two sections: a front vacuum wall section and a rear vacuum wall section, only adjusting the front vacuum wall section, and fixing the rear vacuum wall section. Refer to FIG. 10 and FIG. 11. Specifically, from the distal end to the proximal end of the vacuum wall, the vacuum wall sequentially includes: the front vacuum wall section and the rear vacuum wall section. The front vacuum wall section and the rear vacuum wall section can slide relatively. The vacuum wall adjusting apparatus includes: a sliding block 8, and the sliding block 8 is connected to the front vacuum wall section. The sliding block 8 is configured to move in the axial direction, to drive the front vacuum wall section to move in the axial direction, so that a distal end of the front vacuum wall section is switched between the at least two adjusting positions.

In an embodiment, the mandrel 3 may be used as a second sliding block guiding portion. The vacuum wall is arranged on the outer wall of the mandrel 3. The sliding block 8 is arranged on the outer wall of the vacuum wall. The sliding block 8 moves, and then drives the vacuum wall to move along the mandrel 3. In a different embodiment, the sliding block 8 can also be arranged on the outer wall of the mandrel 3. The sliding block 8 is guided by the mandrel 3 to move in the axis direction. In a different embodiment, a guiding tube arranged in the axis direction of the vacuum wall can also be used for guiding. The guiding tube is arranged in the axis direction. The sliding block 8 is arranged on an inner wall of the guiding tube, slides along the guiding tube, and can also be guided to slide in the axis direction. In a different embodiment, the second sliding block guiding portion may be connected to a component to be adjusted (front vacuum wall section). The sliding block is connected to the first sliding block guiding portion. The first sliding block guiding portion is arranged in the axis direction, and can move in the axis direction. The sliding block 8 drives the first sliding block guiding portion in the axis direction, and then drives the front vacuum wall section to move in the axis direction.

In a preferred embodiment, on the basis of the foregoing embodiment of dividing the vacuum wall into two sections, an outer tube 23 is arranged at the proximal end of the needle rod 21. An outer diameter of the outer tube 23 is greater than an outer diameter of the needle rod 21, and an inner diameter of the outer tube 23 is greater than an inner diameter of the needle rod 21. Front the distal end to the proximal end of the inner tube 22, the inner tube 22 sequentially includes: an inner tube front section 221 and an inner tube rear section 222. An outer diameter of the inner tube rear section 222 is greater than an outer diameter of the inner tube front section 221. An inner diameter of the inner tube rear section 222 is greater than an inner diameter of the inner tube front section 221. The inner tube front section 221 is located inside the needle rod 21. The inner tube rear section 222 is located inside the outer tube 23. In this case, the division mode of the front vacuum wall section and the rear vacuum wall section is as follows: the front vacuum wall section includes: the needle rod 21 and the inner tube front section 221, and the rear vacuum wall section includes: the outer tube 23 and the inner tube rear section 222. In order to improve a sealing effect, the front vacuum wall section further includes: gaskets 24. The gaskets 24 are arranged between an outer wall of a distal end of the inner tube front section 221 and the needle rod 21, and between an outer wall of a proximal end of the inner tube front section 221 and the needle rod 21.

In a preferred embodiment, on the basis of the foregoing embodiment of dividing the vacuum wall into two sections, a dynamic seal is realized between the front vacuum wall section and the rear vacuum wall section through a sealing assembly, to prevent air leakage therebetween. Refer to FIG. 10 and FIG. 11. The sealing assembly includes: a sealing ring 51 and a sealing slot 52. The sealing slot 52 is located between the front vacuum wall section and the rear vacuum wall section. The sealing ring 51 is located between an outer wall of the distal end of the front vacuum wall section and the sealing slot 52. The sealing ring 51 can move axially along the sealing slot with the front vacuum wall section, to realize a dynamic seal. In this embodiment, since the sealing ring 51 is located on an outer surface of the front vacuum wall section, and this area is in a room temperature state, only a common rubber sealing ring needs to be used, and there is no need to worry about sealing failure in a low temperature state.

In a preferred embodiment, on the basis of the foregoing embodiment of dividing the vacuum wall into two sections, in order to facilitate the adjustment of the sliding block 8, the cryoablation tube with the adjustable vacuum wall position is further provided with a shifting block 86 and a handle 9. The handle 9 is provided with a handle adjusting slot 94 therein. Refer to FIG. 10 and FIG. 11. The sliding block 8 is fixed to the outer surface of the front vacuum wall section. The shifting block 86 is connected to the sliding block 8. The shifting block 86 extends from the handle adjusting slot 94 to the outer wall of the handle 9, which is convenient for adjustment. The sliding block 8 can be driven to move in the axial direction by shifting the shifting block 86 to slide in the handle adjusting slot 94. In order to facilitate the fixing of the adjusting position of the distal end of the vacuum wall, the length of the handle adjusting slot 94 can be set according to the position of the distal end of the vacuum wall. When the shifting block 86 is located at the furthest end of the handle adjusting slot 94, the distal end of the vacuum wall is located at the second adjusting position. When the shifting block 86 is located at the nearest end of the handle adjusting slot 94, the distal end of the vacuum wall is located at the first adjusting position.

In a preferred embodiment, in order to better detect the freezing effect of the cryoablation needle, the cryoablation needle with the adjustable vacuum wall position further includes: a temperature measuring wire 14. A distal end of the temperature measuring wire 14 is a temperature measuring point 141, and the distal end of the temperature measuring wire 14 is an end of the temperature measuring wire 14 close to the needle tip 211. Refer to FIG. 3, FIG. 4, FIG. 6, FIG. 7, FIG. 10 and FIG. 11. The temperature measuring point 141 is arranged at the distal end of the J-T slot 1, and used for measuring temperature at the distal end of the J-T slot 1. When being located in the target area, the distal end of the J-T slot 1 is configured to monitor the central temperature of a tumor during freezing and rewarming processes. When being located in the vacuum insulation area, the distal end of the J-T slot 1 is configured to indicate whether pre-purging is in place through the temperature during a pre-purging process. The temperature measuring wire 14 runs along the outer side of the J-T slot 1, and then leads out from the inside of the mandrel 3 to the outside, and glue is poured into the mandrel 3 for sealing. Preferably, the cryoablation needle with the adjustable vacuum wall position further includes a rewarming wire. The position and arrangement of the rewarming wire are consistent with those of the temperature measuring wire, and the rewarming wire is used for achieving a rewarming function. Preferably, the temperature measuring wire and/or the rewarming wire is a T-type enameled thermocouple wire.

In an embodiment, in order to wrap around components such as the gas intake tube and the gas return tube, and make the cryoablation needle cleaner in appearance and more convenient in operation, an outer sleeve 13 is further arranged on an outer wall of the handle 9. Refer to FIG. 3, FIG. 4, FIG. 6, FIG. 7, FIG. 10 and FIG. 11.

In a preferred embodiment, the pre-purging mode, that is, a state that the distal end of the J-T slot 1 is located in the vacuum insulation area 26, can be set to a factory delivery state of the product, and an operator can directly complete the pre-purging of the product through a needling test procedure. After needling test/pre-purging is completed, the product is further adjusted to the freezing mode, that is, the distal end of the J-T slot 1 being located inside the target area 25. After the freezing mode is enabled, since the cryoablation needle is pre-purged, the target area 25 will rapidly cool down to the lowest temperature.

In the description of this specification, description of reference terms such as "an implementation", "an embodiment", "a specific implementation process" or "an example" means including specific features, structures, materials, or characteristics described in the embodiment or example in at least one embodiment or example of the present invention. In this specification, exemplary descriptions of the foregoing terms do not necessarily refer to the same embodiment or example. Furthermore, specific features, structures, materials or characteristics described may be combined in any suitable manner in one or more embodiments or examples.

Finally, it should be noted that the foregoing embodiments are merely intended for describing the technical solutions of the present invention, but not for limiting the present invention. Although the present invention is described in detail with reference to the foregoing embodiments, persons of ordinary skill in the art should understand that they may still make modifications to the technical solutions described in the foregoing embodiments or make equivalent replacements to some or all technical features thereof, without making the essence of the corresponding technical solutions departing from the scope of the technical solutions of the embodiments of the present invention.

## Claims

1. A cryoablation needle with an adjustable vacuum wall position, comprising a vacuum wall, a J-T slot and a vacuum wall adjusting apparatus, wherein
the vacuum wall comprises: a needle rod and an inner tube;
the needle rod is provided with a needle tip at a distal end;
the inner tube penetrates through the needle rod, and a cavity is formed between the inner tube and the needle rod, the cavity being capable of forming a vacuum;
in an axis direction of the vacuum wall, a first preset distance exists between a distal end of the inner tube and the distal end of the needle rod; the distal end of the inner tube is an end of the inner tube close to the needle tip;
the J-T slot penetrates through the inner tube;
the needle tip is capable of being switched between at least two adjusting positions relative to the J-T slot, the at least two adjusting positions comprising: a first adjusting position and a second adjusting position;
in areas in which the vacuum wall is distributed in the axis direction of the vacuum wall, an area in which the cavity is located is a vacuum insulation area, and an area in which the first preset distance exists is a target area;
when the needle tip is located at the first adjusting position, a distal end of the J-T slot is located in the target area; the distal end of the J-T slot is an end of the J-T slot close to the needle tip;
when the needle tip is located at the second adjusting position, the distal end of the J-T slot is located in the vacuum insulation area;
the vacuum wall adjusting apparatus is configured to enable the needle tip to be switched between the at least two adjusting positions by adjusting a position of the needle tip;
the vacuum wall adjusting apparatus comprises: a first sliding block and a first sliding block guiding portion;
the first sliding block and the vacuum wall are capable of being controlled to synchronously move in a direction guided by the first sliding block guiding portion, to switch the needle tip between the adjusting positions; the first sliding block guiding portion is configured to guide the first sliding block and the vacuum wall to move in the axis direction of the vacuum wall;
when the needle tip is located at the first adjusting position, in the axis direction of the vacuum wall, a second preset distance exists between the distal end of the J-T slot and the needle tip, and the second preset distance at least ensures that an ice ball formed by freezing is wrapped around the needle tip; and
when a distal end of the vacuum wall is located at the second adjusting position, in the axis direction of the vacuum wall, a third preset distance exists between the distal end of the J-T slot and a distal end of the vacuum insulation area, and the third preset distance at least ensures that a refrigerant directly returns from the inside of the vacuum insulation area after being sprayed from the J-T slot, the distal end of the vacuum insulation area being an end of the vacuum insulation area close to the needle tip.

2. The cryoablation needle with the adjustable vacuum wall position according to claim 1, wherein the first sliding block guiding portion is arranged in the axis direction of the vacuum wall;
the first sliding block is connected to the vacuum wall, and a position of the first sliding block guiding portion is fixed relative to the sliding block or the J-T slot;
if the position of the first sliding block guiding portion is fixed relative to the sliding block, the first sliding block guiding portion and the J-T slot are capable of sliding relatively; and if the position of the first sliding block guiding portion is fixed relative to the J-T slot, the first sliding block and the first sliding block guiding portion are capable of sliding relatively.

3. The cryoablation needle with the adjustable vacuum wall position according to claim 2, further comprising: a first sealing assembly, wherein the first sealing assembly is hermetically connected to a proximal end of the inner tube; the proximal end of the inner tube is an end of the inner tube far away from the needle tip; and
a dynamic seal is formed between the first sealing assembly and the first sliding block.

4. The cryoablation needle with the adjustable vacuum wall position according to claim 3, wherein the first sealing assembly comprises: a sealing ring, a sealing slot and a sealing press piece, wherein
a distal end of the sealing slot is hermetically fixed to the proximal end of the inner tube, the distal end of the sealing slot being an end of the sealing slot close to the needle tip;
the sealing ring is arranged between the sealing press piece and the first sliding block, and the sealing press piece is arranged between the sealing ring and the sealing slot;
the sealing ring and the sealing press piece are slidably connected to the sealing slot; and
the first sliding block, the sealing ring, and the sealing press piece are capable of being controlled to synchronously move in the axis direction.

5. The cryoablation needle with the adjustable vacuum wall position according to claim 2, further comprising: a spring and a clamping piece, wherein
one end of the spring moves synchronously with the distal end of the J-T slot, and is further connected to the clamping piece; the clamping piece is capable of entering and exiting from a clamped position;
the other end of the spring is fixed relative to the J-T slot;
when the clamping piece is located at the clamped position, the spring is limited by the clamping piece to keep in a deformation state, and a distal end of the vacuum wall is located at the second adjusting position;
the deformation state is a compression state or a tension state; and
when the clamping piece exits from the clamped position, the spring is capable of generating a restoring force for restoring from the deformation state to a natural state, and the restoring force is capable of driving the distal end of the vacuum wall to enter the first adjusting position from the second adjusting position.

6. The cryoablation needle with the adjustable vacuum wall position according to claim 5, further comprising: a first handle, wherein the first handle comprises: a front handle section and a rear handle section;
the front handle section is fixedly connected to the vacuum wall;
a distal end of the rear handle section is inserted into a proximal end of the front handle section, and the two are capable of sliding relatively; and
the front handle section and/or the rear handle section is provided with a limiting ring, and the limiting ring is configured to limit the furthest distance of the vacuum wall moving toward the distal end.

7. The cryoablation needle with the adjustable vacuum wall position according to claim 6, wherein the clamping piece comprises: a hand-held portion and a C-shaped ring, wherein
the hand-held portion is arranged on the C-shaped ring; and
the C-shaped ring is wrapped around an outer wall of the rear handle section.

8. The cryoablation needle with the adjustable vacuum wall position according to any one of claims 1 to 7, wherein the vacuum wall further comprises: an outer tube and a gasket, wherein
a distal end of the outer tube is hermetically connected to a proximal end of the needle rod, a proximal end of the outer tube is hermetically connected to the proximal end of the inner tube, the distal end of the outer tube is an end of the outer tube close to the needle tip, and the proximal end of the outer tube is an end of the outer tube far away from the needle tip;
the gasket is arranged between an outer wall of the inner tube and an inner wall of the needle rod to form a sealed connection;
from the distal end to the proximal end of the inner tube, the inner tube sequentially comprises: an inner tube front section and an inner tube rear section;
the inner tube front section penetrates through the needle rod; the inner tube rear section penetrates through the outer tube; and
if the vacuum wall comprises: a front vacuum wall section and a rear vacuum wall section, the front vacuum wall section comprises the needle rod and the inner tube front section, and the rear vacuum wall section comprises the outer tube and the inner tube rear section.

9. The cryoablation needle with the adjustable vacuum wall position according to claim 8, wherein an outer diameter of the outer tube is greater than an outer diameter of the needle rod, and an inner diameter of the outer tube is greater than an inner diameter of the needle rod; and
an outer diameter of the inner tube rear section is greater than an outer diameter of the inner tube front section; an inner diameter of the inner tube rear section is greater than an inner diameter of the inner tube front section.

10. The cryoablation needle with the adjustable vacuum wall position according to any one of claims 1 to 7, further comprising: a temperature measuring wire, wherein
a distal end of the temperature measuring wire is a temperature measuring point; the distal end of the temperature measuring wire is an end of the temperature measuring wire close to the needle tip; and
the temperature measuring point is arranged at the distal end of the J-T slot, and used for measuring temperature at the distal end of the J-T slot.

11. A cryoablation needle with an adjustable vacuum wall position, comprising a vacuum wall, a J-T slot and a vacuum wall adjusting apparatus, wherein
the vacuum wall comprises: a needle rod and an inner tube;
the needle rod is provided with a needle tip at a distal end;
the inner tube penetrates through the needle rod, and a cavity is formed between the inner tube and the needle rod, the cavity being capable of forming a vacuum;
in an axis direction of the vacuum wall, a first preset distance exists between a distal end of the inner tube and the distal end of the needle rod; the distal end of the inner tube is an end of the inner tube close to the needle tip;
the J-T slot penetrates through the inner tube;
the needle tip is capable of being switched between at least two adjusting positions relative to the J-T slot, the at least two adjusting positions comprising: a first adjusting position and a second adjusting position;
in areas in which the vacuum wall is distributed in the axis direction of the vacuum wall, an area in which the cavity is located is a vacuum insulation area, and an area in which the first preset distance exists is a target area;
when the needle tip is located at the first adjusting position, a distal end of the J-T slot is located in the target area; the distal end of the J-T slot is an end of the J-T slot close to the needle tip;
when the needle tip is located at the second adjusting position, the distal end of the J-T slot is located in the vacuum insulation area;
the vacuum wall comprises: a front vacuum wall section and a rear vacuum wall section, from a distal end to a proximal end of the vacuum wall, the front vacuum wall section and the rear vacuum wall section are sequentially distributed, and the front vacuum wall section and the rear vacuum wall section are capable of moving relatively; the needle tip is located at the front vacuum wall section;
the vacuum wall adjusting apparatus enables the needle tip to be switched between the at least two adjusting positions by adjusting the relative position of the front vacuum wall section and the rear vacuum wall section;
the vacuum wall adjusting apparatus comprises: a second sliding block and a second sliding block guiding portion;
the second sliding block and the front vacuum wall section are capable of being controlled to synchronously move in a direction guided by the second sliding block guiding portion, to switch the needle tip between the adjusting positions; the second sliding block guiding portion is configured to guide the second sliding block and the front vacuum wall section to move in the axis direction of the vacuum wall;
when the needle tip is located at the first adjusting position, in the axis direction of the vacuum wall, a second preset distance exists between the distal end of the J-T slot and the needle tip, and the second preset distance at least ensures that an ice ball formed by freezing is wrapped around the needle tip; and
when a distal end of the vacuum wall is located at the second adjusting position, in the axis direction of the vacuum wall, a third preset distance exists between the distal end of the J-T slot and a distal end of the vacuum insulation area, and the third preset distance at least ensures that a refrigerant directly returns from the inside of the vacuum insulation area after being sprayed from the J-T slot, the distal end of the vacuum insulation area being an end of the vacuum insulation area close to the needle tip.

12. The cryoablation needle with the adjustable vacuum wall position according to claim 11, wherein the second sliding block guiding portion is arranged in the axis direction of the vacuum wall;
the second sliding block is connected to the front vacuum wall section, and a position of the second sliding block guiding portion is fixed relative to the front vacuum wall section or the rear vacuum wall section; and
if the position of the second sliding block guiding portion is fixed relative to the rear vacuum wall section, the second sliding block and the second sliding block guiding portion are capable of sliding relatively; and if the position of the second sliding block guiding portion is fixed relative to the front vacuum wall section, the rear vacuum wall section and the second sliding block guiding portion are capable of sliding relatively.

13. The cryoablation needle with the adjustable vacuum wall position according to claim 12, further comprising: a second sealing assembly, wherein the second sealing assembly is arranged between the front vacuum wall section and the rear vacuum wall section, and is configured to form a dynamic seal between the front vacuum wall section and the rear vacuum wall section.

14. The cryoablation needle with the adjustable vacuum wall position according to claim 12, further comprising: a shifting block and a second handle, wherein the second handle comprises: a handle adjusting slot;
the shifting block is connected to the sliding block, the shifting block is arranged in the handle adjusting slot, and the shifting block extends out of an outer wall of the second handle;
the shifting block is slidably connected to the handle adjusting slot; and
the shifting block and the second sliding block are capable of being controlled to synchronously move in the axis direction.

15. The cryoablation needle with the adjustable vacuum wall position according to any one of claims 11 to 14, wherein the vacuum wall further comprises: an outer tube and a gasket, wherein
a distal end of the outer tube is hermetically connected to a proximal end of the needle rod, a proximal end of the outer tube is hermetically connected to the proximal end of the inner tube, the distal end of the outer tube is an end of the outer tube close to the needle tip, and the proximal end of the outer tube is an end of the outer tube far away from the needle tip;
the gasket is arranged between an outer wall of the inner tube and an inner wall of the needle rod to form a sealed connection;
from the distal end to the proximal end of the inner tube, the inner tube sequentially comprises: an inner tube front section and an inner tube rear section;
the inner tube front section penetrates through the needle rod; the inner tube rear section penetrates through the outer tube; and
if the vacuum wall comprises: a front vacuum wall section and a rear vacuum wall section, the front vacuum wall section comprises the needle rod and the inner tube front section, and the rear vacuum wall section comprises the outer tube and the inner tube rear section.

16. The cryoablation needle with the adjustable vacuum wall position according to claim 15, wherein an outer diameter of the outer tube is greater than an outer diameter of the needle rod, and an inner diameter of the outer tube is greater than an inner diameter of the needle rod; and
an outer diameter of the inner tube rear section is greater than an outer diameter of the inner tube front section; an inner diameter of the inner tube rear section is greater than an inner diameter of the inner tube front section.

17. The cryoablation needle with the adjustable vacuum wall position according to any one of claims 11 to 14, further comprising: a temperature measuring wire, wherein
a distal end of the temperature measuring wire is a temperature measuring point; the distal end of the temperature measuring wire is an end of the temperature measuring wire close to the needle tip; and
the temperature measuring point is arranged at the distal end of the J-T slot, and used for measuring temperature at the distal end of the J-T slot.
